(19) Europäisches Patentamt
European Patent Office
Office européen des brevets

(11) EP 2 628 011 B1

(12) **EUROPEAN PATENT SPECIFICATION**

(45) Date of publication and mention of the grant of the patent:
**21.12.2016 Bulletin 2016/51**

(21) Application number: **11768005.8**

(22) Date of filing: **12.10.2011**

(51) Int Cl.:
***G01N 33/574*** $^{(2006.01)}$ ***G01N 33/50*** $^{(2006.01)}$
***C07D 239/94*** $^{(2006.01)}$

(86) International application number:
**PCT/EP2011/067757**

(87) International publication number:
**WO 2012/049192 (19.04.2012 Gazette 2012/16)**

(54) **ILK GENE AS MARKER FOR ERLOTINIB TREATMENT**

ILK-GEN ALS MARKER FÜR EINE ERLOTINIB-THERAPIE

ILK GENE EN TANT QUE MARQUEUR POUR LE TRAITEMENT AVEC L'ERLOTINIB

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.07.2011 EP 11173835**
**15.10.2010 EP 10187683**

(43) Date of publication of application:
**21.08.2013 Bulletin 2013/34**

(73) Proprietor: **F. Hoffmann-La Roche AG**
**4070 Basel (CH)**

(72) Inventors:
- **AUGUSTIN, Angélique**
  **CH-4112 Flueh (CH)**
- **DUCHATEAU-NGUYEN, Guillemette**
  **F-68400 Riedisheim (FR)**
- **ESSIOUX, Laurent**
  **F-68220 Attenschwiller (FR)**
- **GOLLING, Sabrina**
  **F-68480 Oltingue (FR)**
- **KLUGHAMMER, Barbara**
  **79618 Rheinfelden (DE)**
- **LAMERZ, Jens**
  **77654 Offenburg (DE)**
- **LANGEN, Hanno**
  **79540 Loerrach (DE)**
- **MEISTERMANN, Hélène**
  **F-68400 Riediesheim (FR)**
- **SCHEIBLICH, Stefan**
  **82377 Penzberg (DE)**
- **TZOUROS, Manuel**
  **CH-2502 Biel/Bienne (CH)**

(74) Representative: **Müller-Afraz, Simona**
**F. Hoffmann-La Roche AG**
**Patent Department**
**Grenzacherstrasse 124**
**4070 Basel (CH)**

(56) References cited:
**US-A1- 2006 211 060**

- **B. C. FUCHS ET AL: "Epithelial-to-Mesenchymal Transition and Integrin-Linked Kinase Mediate Sensitivity to Epidermal Growth Factor Receptor Inhibition in Human Hepatoma Cells", CANCER RESEARCH, vol. 68, no. 7, 1 April 2008 (2008-04-01), pages 2391-2399, XP55016042, ISSN: 0008-5472, DOI: 10.1158/0008-5472.CAN-07-2460**
- **THOMSON S ET AL: "Epithelial to mesenchymal transition is a determinant of sensitivity of non-small-cell lung carcinoma cell lines and xenografts to epidermal growth factor receptor inhibition", CANCER RESEARCH, AMERICAN ASSOCIATION FOR CANCER RESEARCH, US, vol. 65, no. 20, 15 October 2005 (2005-10-15), pages 9455-9462, XP002391562, ISSN: 0008-5472, DOI: 10.1158/0008-5472.CAN-05-1058 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- YAUCH ROBERT L ET AL: "Epithelial versus mesenchymal phenotype determines in vitro sensitivity and predicts clinical activity of erlotinib in lung cancer patients", CLINICAL CANCER RESEARCH, THE AMERICAN ASSOCIATION FOR CANCER RESEARCH, US, vol. 11, no. 24 Pt 1, 15 December 2005 (2005-12-15), pages 8686-8698, XP002577918, ISSN: 1078-0432, DOI: 10.1158/1078-0432.CCR-05-1492
- E.-H. TAN ET AL: "A multicentre phase II gene expression profiling study of putative relationships between tumour biomarkers and clinical response with erlotinib in non-small-cell lung cancer", ANNALS OF ONCOLOGY, vol. 21, no. 2, 1 February 2010 (2010-02-01), pages 217-222, XP55016019, ISSN: 0923-7534, DOI: 10.1093/annonc/mdp520
- MCDONALD PAUL C ET AL: "Integrin-linked kinase--essential roles in physiology and cancer biology", JOURNAL OF CELL SCIENCE, CAMBRIDGE UNIVERSITY PRESS, LONDON, GB, vol. 121, no. 19, 1 October 2008 (2008-10-01), pages 3121-3132, XP009155335, ISSN: 0021-9533, DOI: 10.1242/JCS.017996
- DE LUCA A ET AL: "Predictive biomarkers to tyrosine kinase inhibitors for the epidermal growth factor receptor in non-small-cell lung cancer.", CURRENT DRUG TARGETS JUL 2010 LNKD- PUBMED:20388064, vol. 11, no. 7, July 2010 (2010-07), pages 851-864, XP009155361, ISSN: 1873-5592
- ON BEHALF OF TARCEVA CLINICAL BIOMARKER SUBTEAM ET AL: "68 Chemical proteomics profiling of erlotinib in NSCLC cell lines suggests novel mode of action", EUROPEAN JOURNAL OF CANCER. SUPPLEMENT, PERGAMON, OXFORD, GB, vol. 8, no. 7, 1 November 2010 (2010-11-01), page 30, XP027497756, ISSN: 1359-6349, DOI: 10.1016/S1359-6349(10)71773-0 [retrieved on 2010-11-01]
- AUGUSTIN ANGÉLIQUE ET AL: "Quantitative chemical proteomics profiling differentiates erlotinib from gefitinib in EGFR wild-type non-small cell lung carcinoma cell lines.", MOLECULAR CANCER THERAPEUTICS APR 2013, vol. 12, no. 4, April 2013 (2013-04), pages 520-529, ISSN: 1538-8514

## Description

The present invention provides biomarkers that are predictive for the clinical benefit of erlotinib hydrochloride treatment in cancer patients.

A number of human malignancies are associated with aberrant or over-expression of the epidermal growth factor receptor (EGFR). EGF, transforming growth factor-$\alpha$ (TGF-$\alpha$), and a number of other ligands bind to the EGFR, stimulating autophosphorylation of the intracellular tyrosine kinase domain of the receptor. A variety of intracellular pathways are subsequently activated, and these downstream events result in tumor cell proliferation in vitro. It has been postulated that stimulation of tumor cells via the EGFR may be important for both tumor growth and tumor survival in vivo.

Early clinical data with Tarceva®, an inhibitor of the EGFR tyrosine kinase, indicate that the compound is safe and generally well tolerated at doses that provide the targeted effective concentration (as determined by preclinical data). Clinical phase I and II trials in patients with advanced disease have demonstrated that Tarceva® has promising clinical activity in a range of epithelial tumors. Indeed, Tarceva® has been shown to be capable of inducing durable partial remissions in previously treated patients with head and neck cancer, and NSCLC (Non small cell lung cancer) of a similar order to established second line chemotherapy, but with the added benefit of a better safety profile than chemo therapy and improved convenience (tablet instead of intravenous [i.v.] administration). A randomised, double-blind, placebo-controlled trial (BR.21) has shown that single agent Tarceva® significantly prolongs and improves the survival of NSCLC patients for whom standard therapy for advanced disease has failed.

Tarceva® (erlotinib hydrochloride) is a small chemical molecule; it is an orally active, potent, inhibitor of the EGFR tyrosine kinase (EGFR-TKI).

Iressa ™ (gefitinib) is a small chemical molecule; it is an orally active inhibitor of the EGFR tyrosine kinase (EGFR-TKI).

Lung cancer is the major cause of cancer-related death in North America and Europe. In the United States, the number of deaths secondary to lung cancer exceeds the combined total deaths from the second (colon), third (breast), and fourth (prostate) leading causes of cancer deaths combined. About 75% to 80% of all lung cancers are NSCLC, with approximately 40% of patients presenting with locally advanced and/or unresectable disease. This group typically includes those with bulky stage IIIA and IIIB disease, excluding malignant pleural effusions.

The crude incidence of lung cancer in the European Union is 52.5, the death rate 48.7 cases/100000/year. Among men the rates are 79.3 and 78.3, among women 21.6 and 20.5, respectively. NSCLC accounts for 80% of all lung cancer cases. About 90% of lung cancer mortality among men, and 80% among women, is attributable to smoking.

In the US, according to the American Cancer Society, during 2004, there were approximately 173,800 new cases of lung cancer (93,100 in men and 80,700 in women) and were accounting for about 13% of all new cancers. Most patients die as a consequence of their disease within two years of diagnosis. For many NSCLC patients, successful treatment remains elusive. Advanced tumors often are not amenable to surgery and may also be resistant to tolerable doses of radiotherapy and chemotherapy. In randomized trials the currently most active combination chemotherapies achieved response rates of approximately 30% to 40% and a 1-year survival rate between 35% and 40%. This is really an advance over the 10% 1-year survival rate seen with supportive care alone (Shepherd 1999).

Until recently therapeutic options for relapsed patients following relapse were limited to best supportive care or palliation. A recent trial comparing docetaxel (Taxotere) with best supportive care showed that patients with NSCLC could benefit from second line chemotherapy after cisplatin-based first-line regimens had failed. Patients of all ages and with ECOG performance status of 0, 1, or 2 demonstrated improved survival with docetaxel, as did those who had been refractory to prior platinum-based treatment. Patients who did not benefit from therapy included those with weight loss of 10%, high lactate dehydrogenase levels, multi-organ involvement, or liver involvement. Additionally, the benefit of docetaxel monotherapy did not extend beyond the second line setting. Patients receiving docetaxel as third-line treatment or beyond showed no prolongation of survival. Single-agent docetaxel became a standard second-line therapy for NSCLC. Recently another randomized phase III trial in second line therapy of NSCLC compared pemetrexed (Alimta®) with docetaxel. Treatment with pemetrexed resulted in a clinically equivalent efficacy but with significantly fewer side effects compared with docetaxel.

It has long been acknowledged that there is a need to develop methods of individualizing cancer treatment. With the development of targeted cancer treatments, there is a particular interest in methodologies which could provide a molecular profile of the tumor target, (i.e. those that are predictive for clinical benefit). Proof of principle for gene expression profiling in cancer has already been established with the molecular classification of tumor types which are not apparent on the basis of current morphological and immunohistochemical tests. Two separate disease entities were differentiated with differing prognoses from the single current classification of diffuse large B-cell lymphoma using gene expression profiling.

It has been shown that NSCLC patients carrying an activating EGFR mutation in their tumor respond especially well to treatment with tyrosine kinase inhibitors like erlotinib and gefitinib (Paz-Ares et al. 2010). Whereas gefitinib does not seem to confer clinical benefit to an unselected patient population (Thatcher et al. Lancet 2005) erlotinib does (Shepherd et al. NEJM 2005; Cappuzzo et al 2010). Therefore, it is an aim of the present invention to provide expression biomarkers that are predictive for the clinical benefit of erlotinib hydrochloride treatment in cancer patients and are non-predictive for the clinical benefit of gefitinib treatment in cancer patients.

The ECM (Extracellular Matrix) provides the structural framework for the formation of tissues and organs. The ECM binds to substrate adhesion molecules on the surface of cells and influences various intracellular signaling pathways that regulate survival, proliferation, polarity and differentiation. The important families of adhesion molecules that bind to the ECM are the Integrins. Integrins consist of Alpha and Beta-subunits and are composed of large extracellular domains and relatively small cytoplasmic domains. Ligand binding activates signaling cascades that lead to the assembly of a multiprotein complex at the site of cell adhesion to the ECM. These events have two important impacts on the cell: they forge a connection between the ECM and the Actin cytoskeleton, and they alter the fluxes of many intracellular signaling pathways. Three proteins which act as important regulators of Integrin mediated signaling are the ILK (Integrin Linked Kinase) and the adaptor proteins PINCH (Particularly Interesting Cys-His-rich Protein) and Parv (Parvin), among which ILK is the major regulator of Integrin mediated signaling. These molecules form a heterotrimeric complex, which is referred to as the IPP complex. The main function of ILK is to connect Integrins to the cytoskeleton. ILK binds PINCH through its N-terminal domain and binds alpha-Parvin through its C-terminal domain, resulting in formation of PINCH-ILK-alpha-Parvin ternary complexes (IPP complex). The IPP complex functions both as an adaptor between Integrins and the Actin cytoskeleton and as a hub that regulates several signaling pathways.

Wild-type EGFR containing human NSCLC lines show greater sensitivity to erlotinib hydrochloride before EMT (Thomson *et al.* (2005)). Epithelial-mesenchymal transition (EMT) is a phenotypic conversion of biological cells characterized by loss of cell adhesion, repression of E-cadherin expression, and increased cell mobility. Several oncogenic pathways (e.g. peptide growth factors, integrin, etc.) are believed to induce EMT. EMT is a characteristic feature of cells undergoing proliferation, it has been suggested to play a significant role in tumor progression and metastases. Increased expression of integrin-linked kinase is associated with shorter survival in non-small cell lung cancer (Takanami *et al.* (2005)).

The MERIT study is a non-randomized, open-label efficacy marker identification trial for Tarceva in second-line non-small cell lung cancer patients.

The term "biomarker" means a substance used as an indicator of a biological state, i.e. of biological processes or pharmacologic response to a therapeutic interaction. A biomarker indicates a change in expression or state of a protein that correlates with the risk or progression of a disease, or with the susceptibility of the disease to a given treatment.

The term "marker gene" means a gene used to determine if a piece of DNA has been successfully inserted into the host organism.

Gene expression is the process in which information from a gene is used in the synthesis of a functional gene product, e.g. a protein. The term "expression level" means the level at which a particular gene is expressed within a cell, tissue or organism. The term "expression level" in context with proteins reflects the amount of a particular protein present in a cell at a certain time.

"Chemical proteomics" is a mass-spectrometry based affinity chromatography approach that uses immobilized small molecules as bait to capture and identify interacting protein complexes from an entire proteome. It can be applied on kinase inhibitors for instance, to explore their mode of action, or on natural products for target identification.

The term "activation level" reflects the potential of a particular protein to be active in a cell at a certain time. An example are kinases being phosphorylated at a certain site to be able to modify their substrate.

The term "a value representative of an expression level of the IPP complex in tumors of a population of patients deriving no clinical benefit from the treatment" refers to an estimate of a mean expression level of the marker gene in tumors of a population of patients who do not derive a clinical benefit from the treatment. Clinical benefit was defined as either having an objective response or disease stabilization for $\geq$ 12 weeks.

In a particular embodiment, the expression level of the marker gene is determined by microarray technology or other technologies that assess RNA expression levels like quantitative RT-PCR, or by any method looking at the expression level of the respective protein, eg immunohistochemistry (IHC). The construction and use of gene chips are well known in the art. see, U. S. Pat Nos. 5,202,231; 5,445,934; 5,525,464; 5,695,940; 5,744,305; 5,795, 716 and 1 5,800,992. See also, Johnston, M. Curr. Biol. 8:R171-174 (1998); Iyer VR et al., Science 283:83-87 (1999). Of course, the gene expression level can be determined by other methods that are known to a person skilled in the art such as e.g. northern blots, RT-PCR, real time quantitative PCR, primer extension, RNase protection, RNA expression profiling.

The marker gene of the present invention can be combined with other biomarkers to biomarker sets, in particular with EGFR Biomarkers. Biomarker sets can be built from any combination of predictive biomarkers to make predictions about the effect of erlotinib hydrochloride treatment in cancer patients. The biomarkers and biomarkers sets described herein can be used, for example, to predict how patients with cancer will respond to therapeutic intervention with erlotinib hydrochloride.

The term "gene" as used herein comprises variants of the gene. The term "variant" relates to nucleic acid sequences which are substantially similar to the nucleic acid sequences given by the GenBank accession number. The term "substantially similar" is well understood by a person skilled in the art. In particular, a gene variant may be an allele which shows nucleotide exchanges compared to the nucleic acid sequence of the most prevalent allele in the human population. Preferably, such a substantially similar nucleic acid sequence has a sequence similarity to the most prevalent allele of at least 80%, preferably at least 85%, more preferably at least 90%, most preferably at least 95%. The term "variants" is also meant to relate to splice variants.

Techniques for the detection and quantification of gene expression of the genes described by this invention include, but are not limited to northern blots, RT-PCR, real time quantitative PCR, primer extension, RNase protection, RNA expression profiling and related techniques. These techniques are well known to those of skill in the art see e.g. Sambrook J et al., Molecular Cloning: A Laboratory Manual, Third Edition (Cold Spring Harbor Press, Cold Spring Harbor, 2000).

Techniques for the detection of protein expression of the respective genes described by this invention include, but are not limited to immunohistochemistry (IHC).

The biomarkers of the present invention are an individualized therapy for patients with cancer, in particular patients with refractory NSCLC. This individualized therapy will allow treating physicians to select the most appropriate agent out of the existing drugs for cancer therapy, in particular NSCLC. The benefit of individualized therapy for each future patient are: response rates / number of benefiting patients will increase and the risk of adverse side effects due to ineffective treatment will be reduced.

It is to be understood that the terminology employed herein is for the purpose of describing particular embodiments, and is not intended to be limiting. Further, although any methods, devices and materials similar or equivalent to those described herein can be used in the practice or testing of the invention, the preferred methods, devices and materials are now described.

The present invention surprisingly found that the response of a cancer patient to treatment with erlotinib hydrochloride can be predicted by determining the activation level of ILK protein in a tumor sample of a patient. Further, the difference in clinical outcomes can be attributed to differences in the protein interaction profiles of erlotinib and gefitinib, determined by chemical proteomics.

In a certain object the present invention relates to an in vitro method of predicting the response of a NSCLC patient to treatment with erlotinib hydrochloride comprising:

determining the RNA expression level of the ILK gene in a tumor sample of a patient treated with erlotinib hydrochloride and comparing the expression levels of the ILK gene to a value representative of the expression levels of ILK gene in tumors of a population of patients deriving no clinical benefit from the treatment, wherein a lower expression level of ILK gene downregulated in the tumor sample of the patient is indicative for a patient who will derive clinical benefit from the treatment, wherein the cancer patient does not respond to treatment with gefitinib.

In a certain object the present invention relates to a method as described herein, wherein the expression level is determined by microarray technology.

In a certain object the present invention relates to an in vitro method of predicting the response of a NSCLC patient to treatment with erlotinib hydrochloride comprising:

determining the activation level of the of ILK protein, in a tumor sample of a patient treated with erlotinib hydrochloride and comparing the activation levels of the ILK protein to a value representative of the activation level of ILK protein in tumors of a population of patients deriving no clinical benefit from the treatment, wherein a lower activation level of ILK protein in the tumor sample of the patient is indicative for a patient who will derive clinical benefit from the treatment, wherein the cancer patient does not respond to treatment with gefitinib.

In a certain object the present invention relates to an use of the ILK gene for predicting the response of a NSCLC patient to erlotinib hydrochloride treatment.

In a certain object the present invention relates to an use of erlotinib hydrochloride for treating NSCLC, wherein

i) the activation level of the ILK protein in a tumor sample of the patient treated with erlotinib hydrochloride is measured,

ii) the expression level of the ILK gene ise compared to a value representative of the expression levels of ILK gene in tumors of a population of patients deriving no clinical benefit from the treatment, and

iii) erlotinib hydrochloride is administered to the patient having a lower expression level of ILK gene.

In a certain object the present invention relates to a kit for detecting expression level of the ILK gene, the kit comprising i) a compound capable of specifically detecting the expression level of the ILK gene, and ii) instructions for using said kit to predict responsiveness of a patient suffering from NSCLC treatment with erlotinib hydrochloride, wherein decreased expression level of the ILK gene as compared to a reference sample indicates that the patient may benefit from treatment with erlotinib hydrochloride.

In a certain object the present invention relates to a kit as described herein, wherein a cancer patient does not respond to treatment with gefitinib.

In a further object the present invention provides a therapeutic method of treating a NSCLC patient identified by the in vitro method of the present invention. Said therapeutic method comprises administering erlotinib hydrochloride to the patient who has been selected for treatment based on the predictive expression and/or activation pattern of the genes of enclosed SEQ IDs 1,2 and 3.

**SEQ IDs:**

Seq. Id. No. 1 shows the nucleotide sequence of human ILK.

Seq. Id. No. 2 shows the nucleotide sequence of human alpha-parvin.

Seq. Id. No. 3 shows the nucleotide sequence of human PINCH.

**Figures:**

Figure 1: The changes of protein abundance with increasing compound concentration that was significantly changed by erlotinib hydrochloride, gefitinib or both are displayed. The changes are generally in good agreement with the expectations raised from the p-values (see figure 2). However, LIMS1 (PINCH) and M3K1 were only identified and thus quantified in erlotinib hydrochloride displaced samples, while EPHA1, K0564, KC1E, NUD12, SN1L1 were only found in gefitinib.

Figure 2: The adjusted p-values of erlotinib hydrochloride *vs*. the adjusted p-values of gefitinib after log10 transformation are shown (lines indicate 5 % probability of error). Region A show proteins significantly binding to erlotinib hydrochloride and gefitinib, Region B and C show proteins significantly binding to erlotinib hydrochloride and gefitinib, respectively. Proteins in Region D did not show significant binding in neither of the two. This figure can only display proteins identified in both compounds, for this reason the proteins named above which are present in only erlotinib hydrochloride or gefitinib displaced samples are missing here.

Figure 3: Interaction profiles of erlotinib hydrochloride and gefitinib in various NSCLC cell lines. Whereas the relative amount of EGFR is similar in all cell lines, this figure shows the specific enrichment of ILK, alpha-parvin and PINCH in erlotinib hydrochloride bound fractions.

Figure 4: ATP binding site of aligned and overlaid ILK and EGFR crystal protein structures showing potential interactions of erlotinib and gefitinib with the area of the ILK DFG-loop. Oxygen atoms are colored in red, nitrogen in blue, chlorine in green and fluorine in light cyan. Carbon atoms of the erlotinib/EGFR complex (1M17) are colored in salmon, carbon of the gefitinib/EGFR (2ITY) complex in yellow and carbon atoms of ILK are colored in white. Picture was generated with PyMOL. The different substitutions in the erlotinib and gefitinib aniline rings (the 3 position is substituted with chlorine in gefitinib but with acetylene in erlotinib and the 4 position is unsubstituted in erlotinib but substituted with fluorine in gefitinib). The resulting differences in atom proximities suggest that erlotinib is more likely to bind favorably to the ATP binding site of ILK compared to Gefitinib

Figure 5: Erlotinib slows down the transition of H358 by stabilizing E-cadherin. A) Western blot on E-cadherin. Quantified signals of B) E-cadherin, C) ILK and D) PINCH, normalized on total protein content and averaged between two separate experiments.

Figure 6: IPP expression in NSCLC cell lines. A) Western blots of ILK, alpha-parvin, PINCH, E-cadherin, vimentin and GAPDH on EGFR wild-type NSCLC epithelial cell lines H292, H441, H358, H322, and mesenchymal cell lines H460, H1299, H226 and Calu6. B) Quantified Western blot signals normalized on GAPDH levels and averaged to determine the level of expression of the IPP complex.

Figure 7: E-cadherin (CDH1) and ILK expression in patients with versus patients without clinical benefit. Each dot represents the gene signal (log2 transformed) measured in one sample. Blue and red dots correspond to samples from patients with and without clinical benefit, respectively. (Dotplots performed with Partek software).

## Example

The invention will now be further described in the example below, which is intended as an illustration only and does not limit the scope of the invention.

### *Cell culture.*

H292 cells were maintained in RPMI 1640 medium (Gibco-31870) supplemented with 10% FBS and penicillin/streptomycin/glutamine. A pellet of $110^8$ H292 cells was lysed in 2.5 mL buffer (150 mM NaCl (sodium chloride) 1 mM $CaCl_2$ (calcium chloride) $MgCl_2$ (magnesium dichloride), 1% NP40 (nonyl phenoxypolyethoxylethanol), complete EDTA (Ethylendiamin-tetraacetate) free protease inhibitors, 1 mM orthovanadate and 50 mM Hepes). After 10 minutes incubation on ice, the lysates were cleared by centrifugation for 15 minutes at 12,000xg at 4°C.

### *Compound Synthesis and Immobilization.*

Erlotinib hydrochloride and gefitinib have been chemically modified to be covalently linked to epoxy-activated sepharose 6B beads (GE Healthcare). Coupling of the derivatives to epoxy-activated sepharose 6B was done in 50% dimethylformamide (DMF)/0.1 M sodium carbonate ($Na_2CO_3$) pH 11 in presence of 10 mM sodium hydroxide (NaOH) overnight at room temperature in the dark. After 3 washes with 50% dimethylformamide (DMF)/0.1 M sodium carbonate ($Na_2CO_3$), remaining reactive groups were blocked with 1 M ethanolamine. Subsequent washing steps were performed according to the manufacturer's instructions. Binding efficiency was estimated by comparing the optical densities of the compound solutions before and after coupling at the maximal absorbance wavelengths 247 and 330 nm.

### *Competition experiment and Affinity Chromatography.*

500 μg of the respective proteins were preincubated with increasing concentrations (0, 0.0856, 0.2862, 0.957, 3.2, 10.7, 35.78, 119.6 and 400 μM final concentration) of either erlotinib hydrochloride or gefitinib for 1 hour at 4°C under rotation. Then, samples were mixed with equilibrated epoxy-activated Sepharose beads with immobilized erlotinib hydrochloride or gefitinib for 3 hours at 4°C with rotation. Beads were then washed 3 times with lysis buffer and finally resuspended in 100 μL SDS loading buffer, boiled 5 minutes at 85°C and centrifuged 5 minutes at 12,000xg.

### *SDS-PAGE.*

Elution samples were separated on 4-20% Tris Glycine SDS-PAGE (Invitrogen, EC6025BOX). Gels were fixed 1 hour in 40% ethanol 10% acetic acid and stained overnight using Coomassie blue (Invitrogen LC6025).

### *Mass Spectrometry.*

For in-gel digestion, an adapted protocol of Shevchenko *et al.* (1996) was used. Briefly, each lanesof the gel was cut into 9 slices and loaded onto a 96 wells Proxeon plate. Gel pieces were subsequently washed with milliQ water and acetonitrile, reduced in dithiothreitol and alkylated with iodoacetamide. After thorough washing, pieces were incubated with 50 ng trypsin (Promega, V5111) and allowed to digest overnight. Supernatants of the digests were collected, and peptides from the gel pieces extracted with one change of 25 mM ammonium bicarbonate followed by two changes of 5% formic acid. All supernatants were pooled, dried and resuspended in 2% acetonitrile, 5% formic acid and analyzed by LC-MS/MS using a nanoflow liquid chromatograph (Proxeon EASY_nLC) coupled to an electrospray ion trap mass spectrometer (Velos LTQ-Orbitrap, Thermo Scientific). Peptide separation was done on a ReproSil-PUR C18-AQUA 3 μM column coupled to an AQUA 100 microns trap column over a 60 minutes acetonitrile gradient.

### *Sequence Identification*

Raw data files were processed using the SEQUEST search algorithm 27 (SEQUEST version 27.0, revision 12, Thermo Electron). Searches were performed against the SwissProt Human database (version 48, 219024 entries) with a mass tolerance of +/-10 ppm for parent ions and +/-1.0 Da for fragment ions. Methionines (reduced/oxidized; +15.9949 Da) were considered as differential modifications while cysteines were considered as fully carbamidomethylated (+58.0133 Da). Only fully tryptic peptides with no more than one miscleavage were considered for data analysis. Peptides were considered as unambiguously identified if their XCorr scores exceeded 2.0, 2.5 or 3.0 for singly, doubly and triply charged ions respectively and if the corresponding dCn scores were larger than 0.1. In this report, only proteins for which at least two different peptides were identified were further processed for quantification purposes.

***Mass spectrometric data processing by Genedata RefinerMS 5.2.6***

For each compound and each band independently, the respective Thermo raw files were loaded by Genedata RefinerMS 5.2.6: Centroid data are discarded to reduce memory usage. Signals occuring in only one spectrum are removed; all data representations of samples (=chromatograms) are given a common 2D m/z-RT grid. Before Chromatogram Alignment which relies on appropriate peak finding parameters, peak finding parameters were visually inspected. Chromatogram Alignment corrects for retention time shifts between chromatograms, based on found common peaks and Trivial Tree Algorithm. The success of this operation was visually inspected. RT Range Restriction discards signals and data outside the information rich region of 23-52 min of chromatography. The Chromatogram Average activity computes a virtual average chromatogram of all input chromatograms. The level of noise is reduced by the "Intensity Threshold" workflow step, eliminating each signal below a threshold of 1000 au. Peaks and their boundaries in RT and m/z direction are found in, and visualized as peak-surrounding squares. The squares are projected on each chromatogram, and the eXtracted Ion Counts (XIC) are determined within these boundaries. The resulting spreadsheet with one row per peak and one column per chromatogram is saved as file in the next workflow step. The ScanID's of MS/MS scans found within these signal boundaries are saved in the same way. A report is generated that summarizes the workflow steps, their order parameters plus diagnostics information from the steps (e.g. degree of local chromatographic shift per sample, number of signals found).

***Further Processing***

For each compound and each band independently, the respective samples were normalized using the Quantile approach (Smyth 2003). The peaks found in RefinerMS are assigned with the sequence information via their ScanID in the respective measurement. Peaks without identification or with more ambiguous identification are removed. The quantitative information is log2-transformed. For Process Control, quantitative data is subjected to Principal Component Analysis: Scores of the first 4 principal components were correlated with Compound Concentration and process parameters, such as gel lane number, LC-MS Batch ID or LC-MS run Number. Mahalanobis Distance outliers are estimated based on the scores of the first two principal components; samples outside the 95 % confidence interval were considered as outliers and removed from further analysis.

***Combination of Peptide intensities to Protein Surrogate***

Peptides are assigned to their potential protein precursors. Each found combination of protein precursors is given a separate protein quantitation group, typically with only one protein member, or a protein and its splice variants. Following Irizarry *et al.* (2003), a relative abundance for each protein quantitation group is obtained from the normalized and log-transformed eXtracted Ion Counts (XIC), fitting a linear additive model:

$$\mathrm{XIC}_{ijn} = \mu_{in} + \alpha_{jn} + \varepsilon_{ijn},$$

with $\alpha_j$ the peptide effect, $\mu_i$, the log scale relative abundance of the protein quantitation group, and $\varepsilon_{ij}$ representing an independent identically distributed error term with mean 0. As described in Irizarry *et al.* (2003) and Holder *et al.* (2001), the median polish is employed to robustly estimate model parameters.

***Detecting significant Dose Response***

A Hirotsu contras matrix is derived from the number compound concentrations (see Bretz *et al.* (2001)), the first and last contrast are eliminated. Moderated t-statistics $T^{SC}$ are obtained for each contrast and protein quantitation group (see Smyth *et al.* (2004)). For each protein quantitation group, $T^{MC}$ = max ($T^{SC}$) is obtained (see Bretz *et al.* (2001)). P-values are obtained based on the algorithm for step-down maxT adjusted p-values with 1000 permutations based on Ge *et al.* (2003), which originates from Westfall and Young (1993). It is modified with respect to the one-sidedness of the statistic by not choosing |T| but T.

Visualizations and any computations are performed with R 2.10.1 (citation "R").

| Protein | Signal Reduction (%) Gefitinib | Erlotinib hydrochloride |
|---|---|---|
| ILK | 0.0 | 77.3 |
| PINCH | 0.0 | 87.7 |
| alpha-parvin | 0.0 | 79.5 |

***Confirmation of binding profiles in other NSCLC cell lines***

H292, H441, H460 and H1299 cells were maintained in RPMI 1640 medium supplemented with 10% fetal bovine serum and 2 mmol/L L-glutamine. Compound synthesis, immobilization and affinity chromatography were performed as described above. Mass spectrometry analysis was performed on an LC system (LC-Packings) connected to a Thermo Finnigan LCQ Deca XP was used. Briefly, peptide extracts (in 5% acetonitrile, 0.5% acetic acid, 0.012% HFBA (heptafluorobutyric acid) with 1% formic acid) were injected on a trap column (PepMap™ C18, 5 μm (LC-Packings), 5 mm x 300 μm I.D.) at 20 μL/min. Subsequently, the peptides were transferred in solvent A (5% acetonitrile, 0.5% acetic acid, 0.012% HFBA) on the analytical column (Magic C18™ 5 μm (Spectronex), 10 cm x 75 μm I.D.). Elution was performed with a 3 step linear gradient from 5 to 15% solventB (0.5% acetic acid, 0.012% HFBA in 80% (v/v) acetonitrile) in 8 min, from 15 to 38% solvent B in 65 min and from 38 to 80% solvent B in 25 min. The column effluent was directly introduced into the ESI source of the mass spectrometer. The mass spectrometer was operated in the positive ion mode and parent ions were selected for fragmentation in data-dependent mode. Raw MS/MS data were searched against human SwissProt database using SEQUEST (version 27). Parameters included one missed cleavage allowed and a peptide mass tolerance of 1.5 Da.

A model NSCLC cell line, H358, was used, which had been previously described as switching from epithelial to mesenchymal state upon TGFbeta3 treatment (Thomson 2008). The results show a decrease of E-cadherin after 13 days of TGFbeta3 treatment, indicating that the cells are actually transitioning (Figure 5a). The level of E-cadherin in H358 cells treated with TGFbeta3 and erlotinib remained unchanged, unlike in cells treated with TGFbeta3 and gefitinib. The quantification of the western blot signals shows a decrease of more than 1/3 in E-cadherin levels (Figure 6b) between non treated cells and cells treated for 13 days with TGFbeta3 . The treatment with erlotinib prevents this decrease, while gefitinib fails to inhibit the TGFb3-mediated decrease of E-cadherin levels. (Figure 5b). The effects on ILK and PINCH expression shows that the levels of both proteins increase after TGFb3 treatment (Figure 5 c and d). The ILK and PINCH expression levels do not change when the cells are treated with erlotinib, an effect which was not so pronounced upon gefitinib treatment. This EMT model therefore, demonstrates that erlotinib reduces the extent of EMT by inhibiting the loss of E-cadherin and also suggests to prevent a TGFb3-mediated increase of ILK and PINCH.

The three components of the complex ILK, alpha-parvin and PINCH are studied on an individual basis, not addressing the possibility of the expression of the entire complex being correlated to an EMT status. Eight EGFR wild type NSCLC cell lines were selected depending on this status (Thomson 2005; Thomson 2008), which was assessed by the expression of E-cadherin as an epithelial marker and vimentin as a mesenchymal marker. All the IPP proteins were detected with various intensities in all eight cell lines except for alpha-parvin in H358 (Figure 6a). After quantification and normalization, the overall sum of IPP complex intensities was 1.8 times higher in mesenchymal versus epithelial cells. PINCH1 was the most highly protein (56% and 58% of total IPP expression in epithelial and mesenchymal cells respectively), followed by ILK (25% and 22%) alpha-parvin (19% and 20%) (Figure 6b).

***E-cadherin and ILK expression***

The potential relationship between IPP and more global genes involved in EMT have been evaluated. Specifically the differences between their expression profiles in patients with and without clinical benefit to erlotinib. In total, only 16 out of the 21 pre-selected genes were identified with detected probe sets and were statistically analyzed. Among the 31 probe sets (corresponding to 16 unique genes), two were statistically significant differentially expressed in tumor samples from patients with and without clinical benefit. The two corresponding genes were ILK and CDH1 (E-cadherin), which are downregulated (0.93-fold, p=0.011) and upregulated (1.38-fold, p=0.033) respectively in patients with clinical benefit.

**Alignment of EGFR and ILK crystal protein structures**

Publicly available crystal structure pdb files of EGFR-bound erlotinib or gefitinib and the crystal structure of ILK complexed with adenosine triphosphate (ATP) were downloaded from the crystallographic database PDB (Berman 2000) (pdb codes 1M17, 2ITY, 3KMW; Stamos 2002; Yun 2007; Fukuda 2009). The three kinase structures were structurally aligned using the coordinates from Cα atoms of a subset of the residues forming the ATP binding site (residue numbers based on EGFR numbering 691-693, 701-703, 719-72 (19) 1, 751, 766-772, 820-823, 831-832. Alignment and distance measurements were done with the PyMOL visualization package.

**Testing EMT transition on H358 NSCLC cell line**

H358 cells were treated with recombinant 10ng/mL TFGβ3 (Peprotech, New York, NY, USA) and erlotinib or gefitinib 5 μM for 3, 6, 10 and 13 days. Cell culture medium was renewed every 3 days. Cell lysis was performed in PBS containing 1% NP-40 and protease inhibitors. After clearing by centrifugation at 12,000g for 15 min at 4 °C, proteins were separated by SDS-PAGE and processed for western blot analysis. Images were acquired using a compact digital camera (Luminescent Image Analyzer: Fujifilm, Tokyo, Japan) and quantified with ImageQuant TL (GE Healthcare, Waukesha, WI, USA).

**Analysis of MERIT data**

Gene expression profiles were measured in tumor biopsy samples from 102 patients with stage IIIb/IV NSCLC, being treated with erlotinib (150 mg/day). Tumor biopsies were analyzed using gene expression profiling with Affymetrix GeneChip Human Genome U133A 2.0 Array (Affymetrix, Santa Clara, CA, USA). All gene expression results described here (fold changes and *p* values) come from the statistical analysis already reported (Tan 2010). We specifically looked at differences between gene expression profiles of patients with and without clinical benefit from erlotinib. The following 21 proteins, potentially involved in transcriptional regulation of EMT or in IPP complex, were mapped to the corresponding genes and microarray probe sets: Twist, Snail [Sma1], Zeb [TCF8], Lef-1, E12/E47 basic Loop helix [bHLH], Slug [Sma2], E-cadherin [CDH1], N-cadherin [CDH2], Myc, Vimentin, Catenin beta, Catenin delta, Fibronectin, Epimorphin, integrin-linked kinase [ILK], Parvin alpha, Parvin beta, PINCH 1, PINCH 2, Metastasis associated protein 3 [MTA3], Y box binding protein 1 [YB-1]).

**References**

Shevchenko, A.; Wilm, M.; Vorm, O.; Mann, M. Mass spectrometric sequencing of proteins silver-stained polyacrylamide gels. Anal Chem 1996; 68, 850-58.

Smyth, G. K., and Speed, T. P. (2003). Normalization of cDNA microarray data. Methods 31, p265-273.

Smyth, G. K., Michaud, J., and Scott, H. (2005). The use of within-array replicate spots for assessing dierential expression in microarray experiments. Bioinformatics 21(9), 2067-2075.

Irizarry RA, Hobbs B, Collin F, Beazer-Barclay YD, Antonellis KJ, Scherf U, Speed TP. (2003) Exploration, normalization, and summaries of high density oligonucleotide array probe level data. Biostatistics Apr;4(2):249-64.

Holder, D, Raubertas, RF, Pikounis, VB, Svetnik, V and Soper, K (2001). Statistical analysis of high density oligonucleotide arrays: a SAFER approach. Proceedings of the ASA Annual Meeting 2001. Atlanta,GA.

Bretz F, Hothorn LA. (2001) Testing dose-response relationships with a priori unknown, possibly nonmonotone shapes. J Biopharm Stat. 11(3):193-207.

Smyth GK. (2004) Linear models and empirical bayes methods for assessing differential expression in microarray experiments. Stat Appl Genet Mol Biol.; 3:Article3. Epub 2004 Feb 12.

Bretz F, Hothorn LA. (2002) Detecting dose-response using contrasts: asymptotic power and sample size determination for binomial data. Stat Med. 2002 21(22):3325-35.

P. H. Westfall and S. S. Young (1993). Resampling-based multiple testing: Examples and methods for p-value adjustment. John Wiley & Sons, Algorithm 4.1, p116-117.

R Development Core Team (2009). R: A language and environment for statistical computing. R Foundation for Statistical Computing, Vienna, Austria. ISBN 3-900051-07-0, URL http://www.R-project.org.

Ge,Y. et al. (2003) Resampling-based multiple testing for microarray data analysis, Test, 12(1), 1-77.

Thomson et al.; 2005; Cancer Res.; 65; 9455-9462.

Takanami, I.; 2005; BMC Cancer; 5; 1-7.

Berman, H. M., Westbrook, J., Feng, Z., Gilliland, G., Bhat, T. N., Weissig, H., Shindyalov, I. N., and Bourne, P. E. (2000) The Protein Data Bank. pp. 235-242.

Stamos, J., Sliwkowski, M. X., and Eigenbrot, C. (2002) Structure of the epidermal growth factor receptor kinase domain alone and in complex with a 4-anilinoquinazoline inhibitor. J Biol Chem 277, 46265-46272.

Yun, C. H., Boggon, T. J., Li, Y., Woo, M. S., Greulich, H., Meyerson, M., and Eck, M. J. (2007) Structures of lung cancer-derived EGFR mutants and inhibitor complexes: mechanism of activation and insights into differential inhibitor sensitivity. Cancer Cell 11, 217-227.

Fukuda, K., Gupta, S., Chen, K., Wu, C., and Qin, J. (2009) The pseudoactive site of ILK is essential for its binding to alpha-Parvin and localization to focal adhesions. Mol Cell 36, 819-830.

Tan, E. H., Ramlau, R., Pluzanska, A., Kuo, H. P., Reck, M., Milanowski, J., Au, J. S., Felip, E., Yang, P. C., Damyanov, D., Orlov, S., Akimov, M., Delmar, P., Essioux, L., Hillenbach, C., Klughammer, B., McLoughlin, P., and Baselga, J. (2010) A multicentre phase II gene expression profiling study of putative relationships between tumour biomarkers and clinical response with erlotinib in non-small-cell lung cancer. Ann Oncol 21, 217-222.

Yauch, R. L., Januario, T., Eberhard, D. A., Cavet, G., Zhu, W., Fu, L., Pham, T. Q., Soriano, R., Stinson, J., Seshagiri, S., Modrusan, Z., Lin, C. Y., O'Neill, V., and Amler, L. C. (2005) Epithelial versus mesenchymal phenotype determines in vitro sensitivity and predicts clinical activity of erlotinib in lung cancer patients. Clin Cancer Res 11, 8686-8698.

Cabodi, S., Del Pilar Camacho-Leal, M., Di Stefano, P., and Defilippi, P. (2010) Integrin signalling adaptors: not only figurants in the cancer story. Nat Rev Cancer 10, 858-870.

Thomson, S., Buck, E., Petti, F., Griffin, G., Brown, E., Ramnarine, N., Iwata, K. K., Gibson, N., and Haley, J. D. (2005) Epithelial to mesenchymal transition is a determinant of sensitivity of non-small-cell lung carcinoma cell lines and xenografts to epidermal growth factor receptor inhibition. Cancer Res 65, 9455-9462.

Thomson, S., Petti, F., Sujka-Kwok, I., Epstein, D., and Haley, J. D. (2008) Kinase switching in mesenchymal-like non-small cell lung cancer lines contributes to EGFR inhibitor resistance through pathway redundancy. Clin Exp Metastasis 25, 843-854.

Singh, A., and Settleman, J. (2010) EMT, cancer stem cells and drug resistance: an emerging axis of evil in the war on cancer. Oncogene 29, 4741-4751.

Oloumi, A., McPhee, T., and Dedhar, S. (2004) Regulation of E-cadherin expression and beta-catenin/Tcf transcriptional activity by the integrin-linked kinase. Biochim Biophys Acta 1691, 1-15.

## Claims

1. An in vitro method of predicting the response of a NSCLC patient to treatment with erlotinib hydrochloride comprising:

   determining the RNA expression level of the ILK gene in a tumor sample of a patient treated with erlotinib hydrochloride and comparing the expression levels of the ILK gene to a value representative of the expression levels of ILK gene in tumors of a population of patients deriving no clinical benefit from the treatment, wherein a lower expression level of ILK gene downregulated in the tumor sample of the patient is indicative for a patient who will derive clinical benefit from the treatment, wherein the cancer patient does not respond to treatment with gefitinib.

2. The method according to claim 1, wherein the expression level is determined by microarray technology.

3. Use of the ILK gene for predicting the response of a NSCLC patient to erlotinib hydrochloride treatment, wherein the NSCLC patient does not respond to treatment with gefitinib.

4. Erlotinib hydrochloride for use in treating NSCLC, wherein

   i) the activation level of ILK protein in a tumor sample of a patient treated with erlotinib hydrochloride is measured,

ii) the RNA expression level of the ILK gene is compared to a value representative of the expression level of ILK gene in tumors of a population of patients deriving no clinical benefit from the treatment, and
iii) erlotinib hydrochloride is administered to a patient having a lower expression level of ILK gene,

wherein the NSCLC patient does not respond to treatment with gefitinib.

**Patentansprüche**

1. *In vitro*-Verfahren zur Vorhersage der Reaktion eines NSCLC-Patienten auf die Behandlung mit Erlotinib-Hydrochlorid, umfassend:

   Bestimmen des RNA-Expressionsspiegels des ILK-Gens in einer Tumorprobe eines Patienten, der mit Erlotinib-Hydrochlorid behandelt wird, und Vergleichen der Expressionsspiegel des ILK-Gens mit einem Wert, der für die Expressionsspiegel des ILK-Gens in Tumoren einer Population von Patienten repräsentativ ist, die keinen klinischen Nutzen aus der Behandlung ziehen, wobei ein niedriger Expressionsspiegel des ILK-Gens, der in der Tumorprobe des Patienten herunterreguliert ist, auf einen Patienten hinweist, der aus der Behandlung einen klinischen Nutzen ziehen wird, wobei der Krebspatient auf eine Behandlung mit Gefitinib nicht anspricht.

2. Verfahren nach Anspruch 1, wobei der Expressionsspiegel durch Microarray-Technologie bestimmt wird.

3. Verwendung eines ILK-Gens zur Vorhersage der Reaktion eines NSCLC-Patienten auf die Behandlung mit Erlotinib-Hydrochlorid, wobei der NSCLC-Patient auf die Behandlung mit Gefitinib nicht anspricht.

4. Erlotinib-Hydrochlorid zur Verwendung bei der Behandlung von NSCLC, wobei

   (i) der Aktivierungsspiegel des ILK-Proteins in einer Tumorprobe eines Patienten, der mit Erlotinib-Hydrochlorid behandelt wird, gemessen wird,
   (ii) der RNA-Expressionsspiegel des ILK-Gens mit einem Wert verglichen wird, der für den Expressionsspiegel eines ILK-Gens in Tumoren einer Population von Patienten repräsentativ ist, die keinen klinischen Nutzen aus der Behandlung ziehen, und
   (iii) Erlotinib-Hydrochlorid an einen Patienten verabreicht wird, der einen niedrigeren Expressionsspiegel des ILK-Gens hat,

   wobei der NSCLC-Patient auf die Behandlung mit Gefitinib nicht anspricht.

**Revendications**

1. Procédé in vitro de prévision de la réponse d'un patient atteint d'un CBNPC à un traitement avec du chlorhydrate d'erlotinib comprenant :

   la détermination du niveau d'expression de l'ARN du gène ILK dans un échantillon de tumeur d'un patient traité avec le chlorhydrate d'erlotinib et la comparaison des niveaux d'expression du gène ILK à une valeur représentative des niveaux d'expression du gène ILK dans les tumeurs d'une population de patients ne tirant aucun avantage clinique du traitement, dans lequel un niveau d'expression inférieur du gène ILK régulé négativement dans l'échantillon de tumeur du patient est indicatif d'un patient qui tirera un avantage clinique du traitement, **caractérisé en ce que** le patient atteint d'un cancer ne répond pas au traitement par géfitinib

2. Procédé selon la revendication 1, dans lequel le niveau d'expression est déterminé par une technologie à micro-puce.

3. Utilisation du gène ILK pour prévoir la réponse d'un patient atteint d'un CBNPC à un traitement de chlorhydrate d'erlotinib, **caractérisé en ce que** le patient atteint d'un CBNPC ne répond pas au traitement par géfitinib.

4. Chlorhydrate d'erlotinib pour une utilisation dans le traitement du CBNPC, **caractérisé en ce que**

   i) le niveau d'activation d'une protéine ILK dans un échantillon de tumeur d'un patient traité avec du chlorhydrate d'erlotinib est mesuré,

ii) le niveau d'expression de l'ARN du gène ILK est comparé à une valeur représentative du niveau d'expression du gène ILK dans des tumeurs d'une population de patients ne tirant aucun bénéfice clinique du traitement, et
iii) le chlorhydrate d'erlotinib est administré à un patient ayant un niveau d'expression inférieur du gène ILK,

**caractérisé en ce que** le patient atteint d'un CBNPC ne répond pas au traitement par géfitinib.

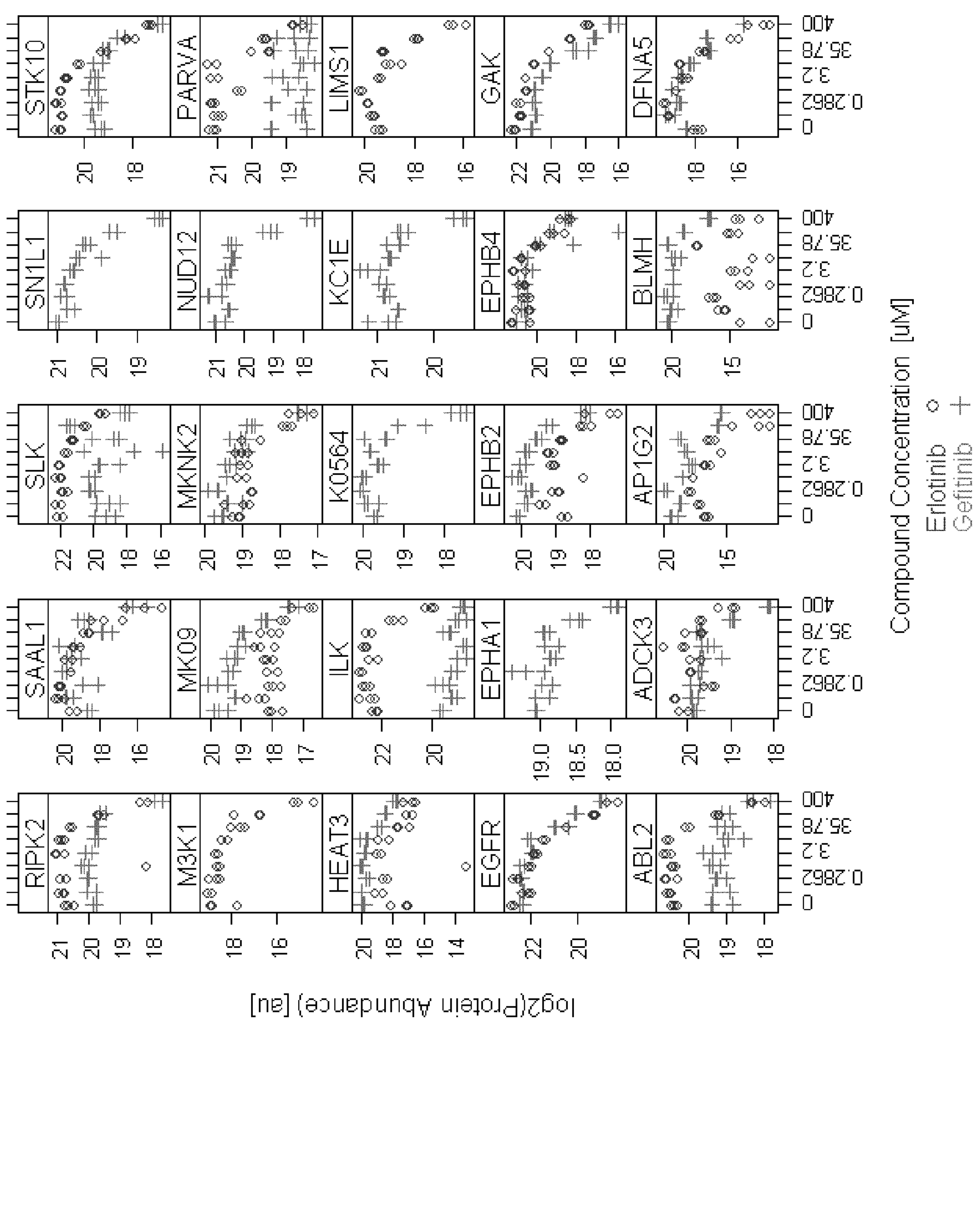
RIPK2
SAAL1
SLK
SN1L1
STK10
M3K1
MK09
MKNK2
NUD12
PARVA
HEAT3
ILK
K0564
KC1E
LIMS1
EGFR
EPHA1
EPHB2
EPHB4
GAK
ABL2
ADCK3
AP1G2
BLMH
DFNA5
log2(Protein Abundance) [au]
Compound Concentration [uM]
Erlotinib
Gefitinib
0
0.2862
3.2
35.78
400

Figure 1

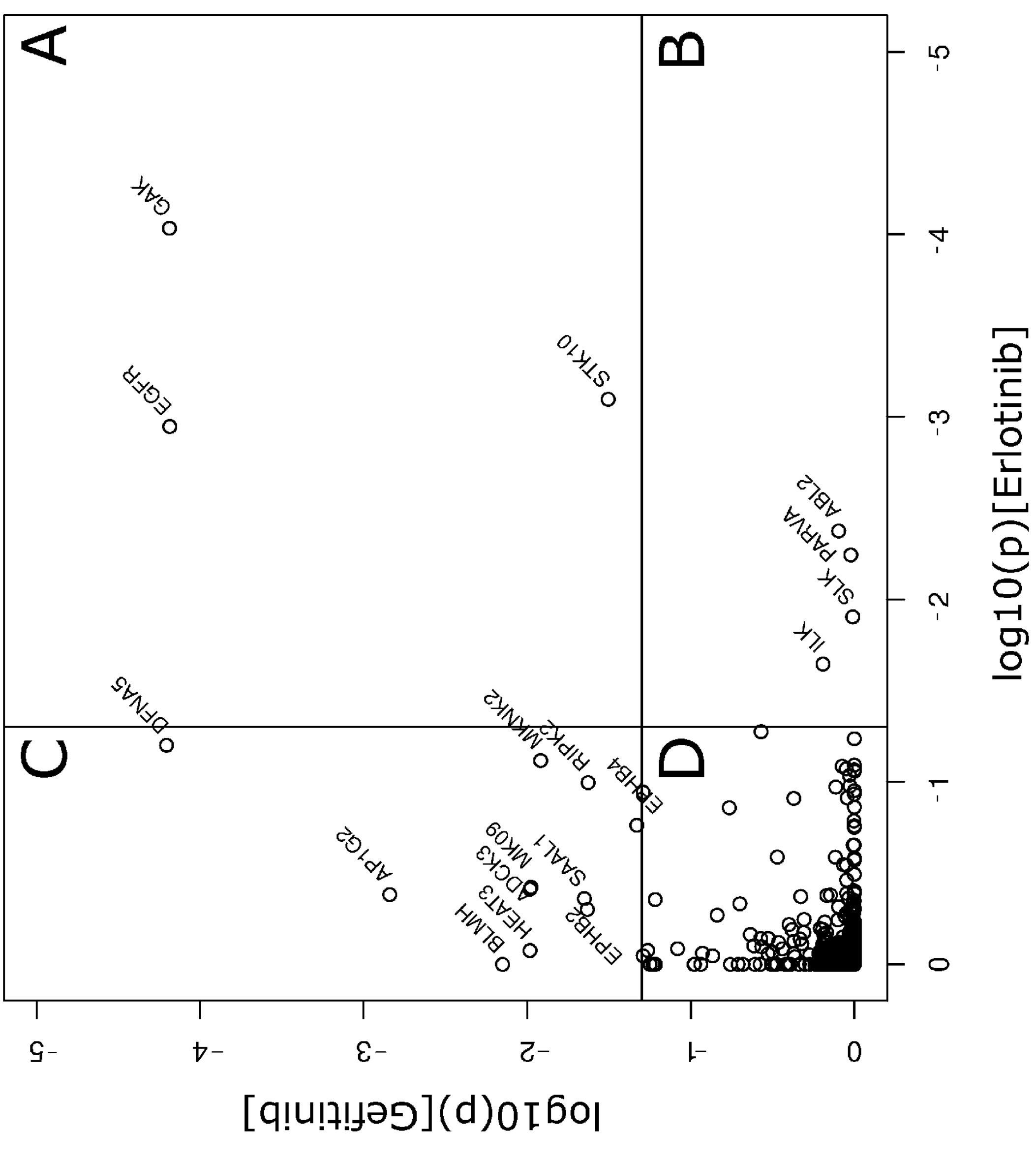
A
B
C
D
GAK
EGFR
STK10
DFNA5
MKNK2
RIPK2
EPHB4
AP1G2
MK09
ADCK3
HEAT3
BLMH
SAAL1
EPHB2
ILK
SLK
PARVA
ABL2
0
-1
-2
-3
-4
-5
log10(p)[Erlotinib]
log10(p)[Gefitinib]

Figure 2

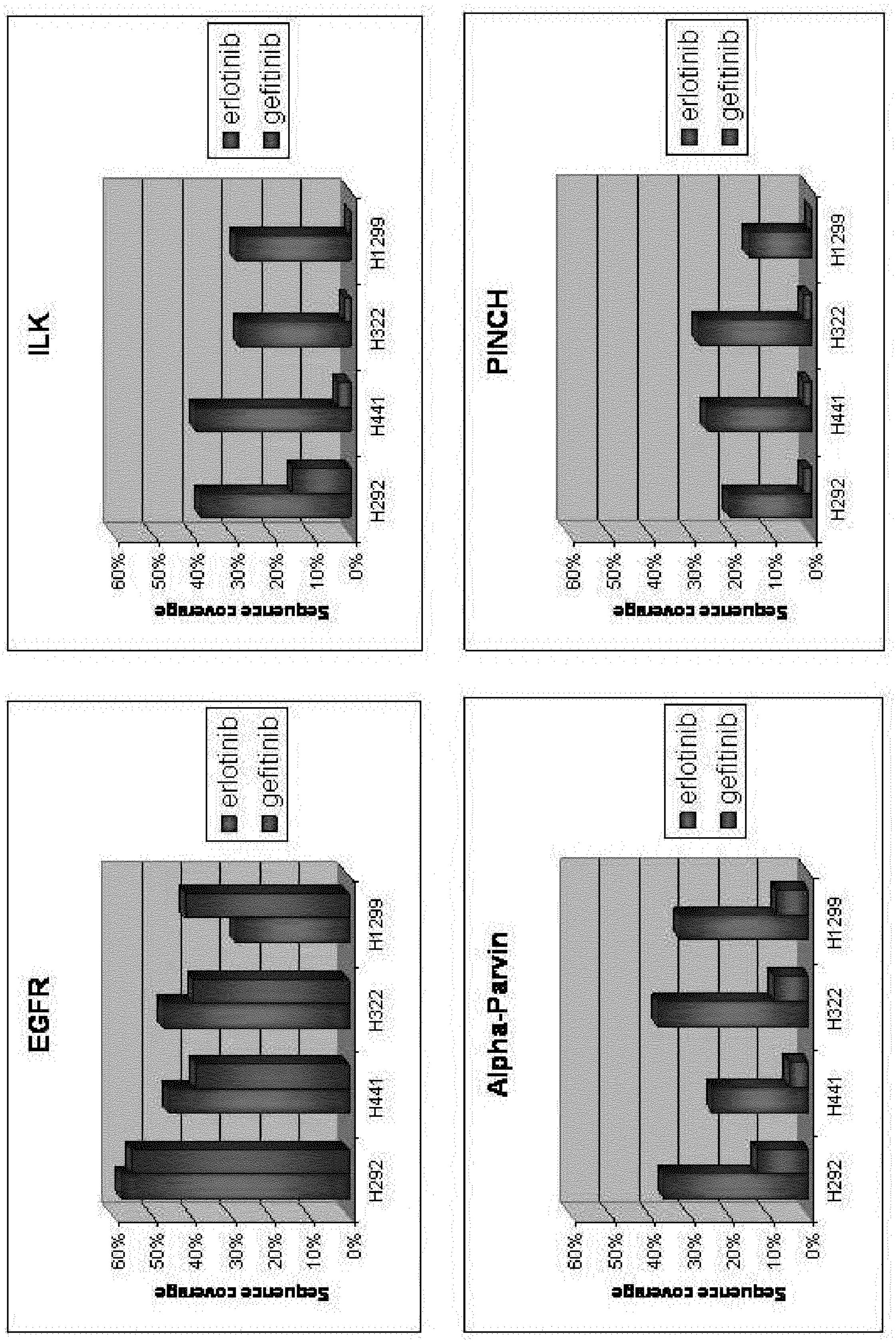
EGFR
ILK
Alpha-Parvin
PINCH
Sequence coverage
60%
50%
40%
30%
20%
10%
0%
H292
H441
H322
H1299
erlotinib
gefitinib

Figure 3

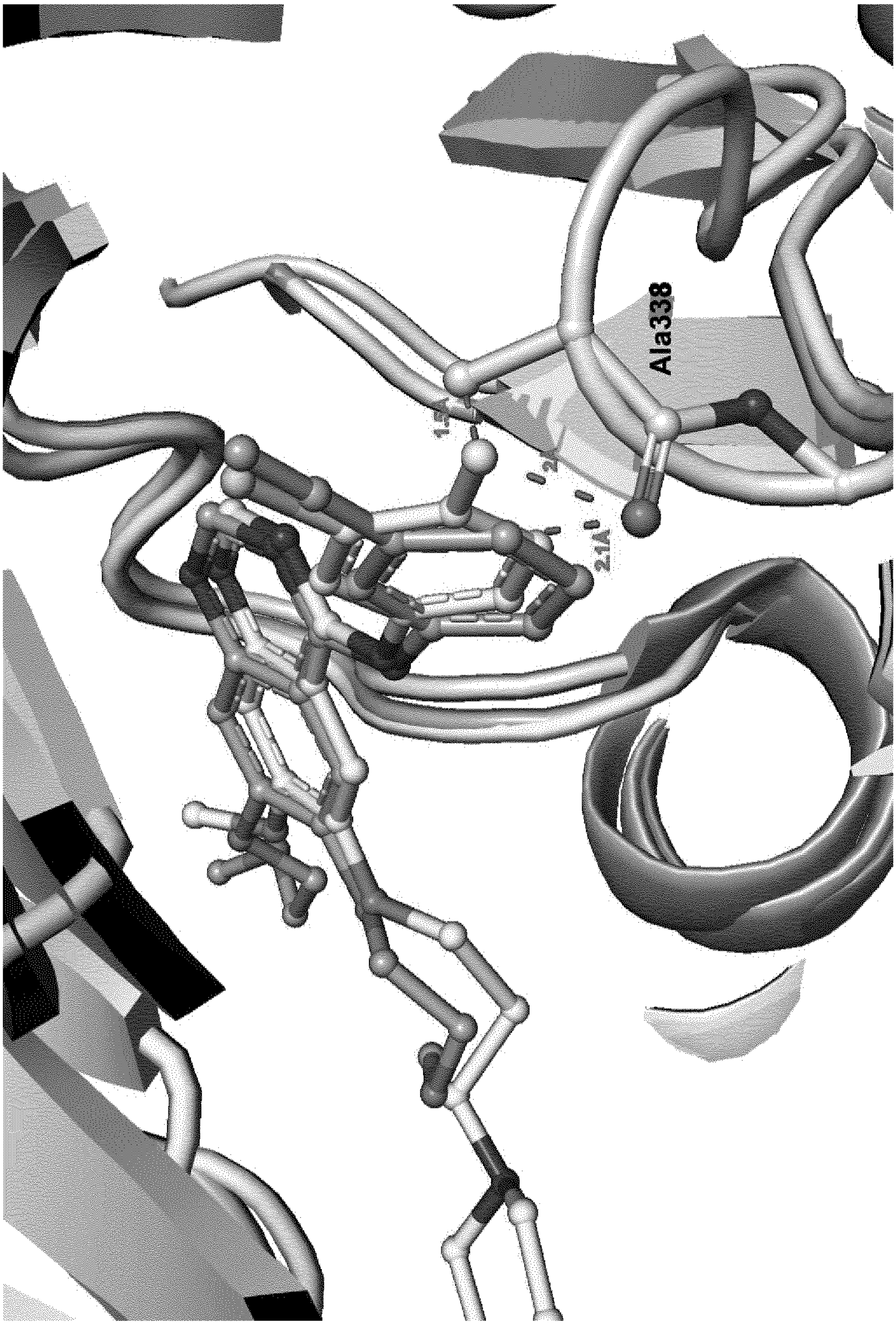


Figure 4

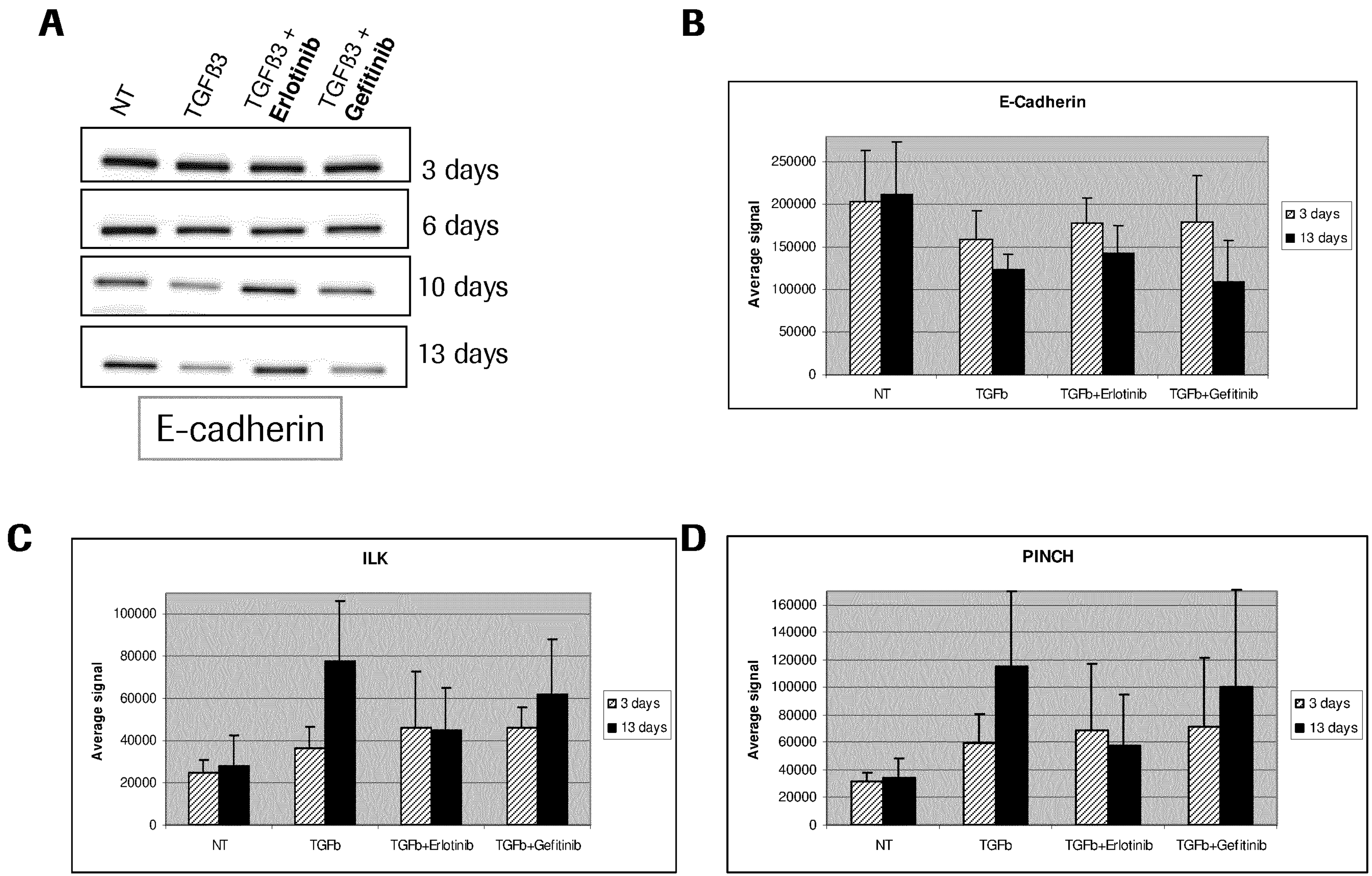
A
NT
TGFβ3
TGFβ3 + Erlotinib
TGFβ3 + Gefitinib
3 days
6 days
10 days
13 days
E-cadherin
B
E-Cadherin
Average signal
250000
200000
150000
100000
50000
0
NT
TGFb
TGFb+Erlotinib
TGFb+Gefitinib
3 days
13 days
C
ILK
Average signal
100000
80000
60000
40000
20000
0
NT
TGFb
TGFb+Erlotinib
TGFb+Gefitinib
3 days
13 days
D
PINCH
Average signal
160000
140000
120000
100000
80000
60000
40000
20000
0
NT
TGFb
TGFb+Erlotinib
TGFb+Gefitinib
3 days
13 days

Fgure 5

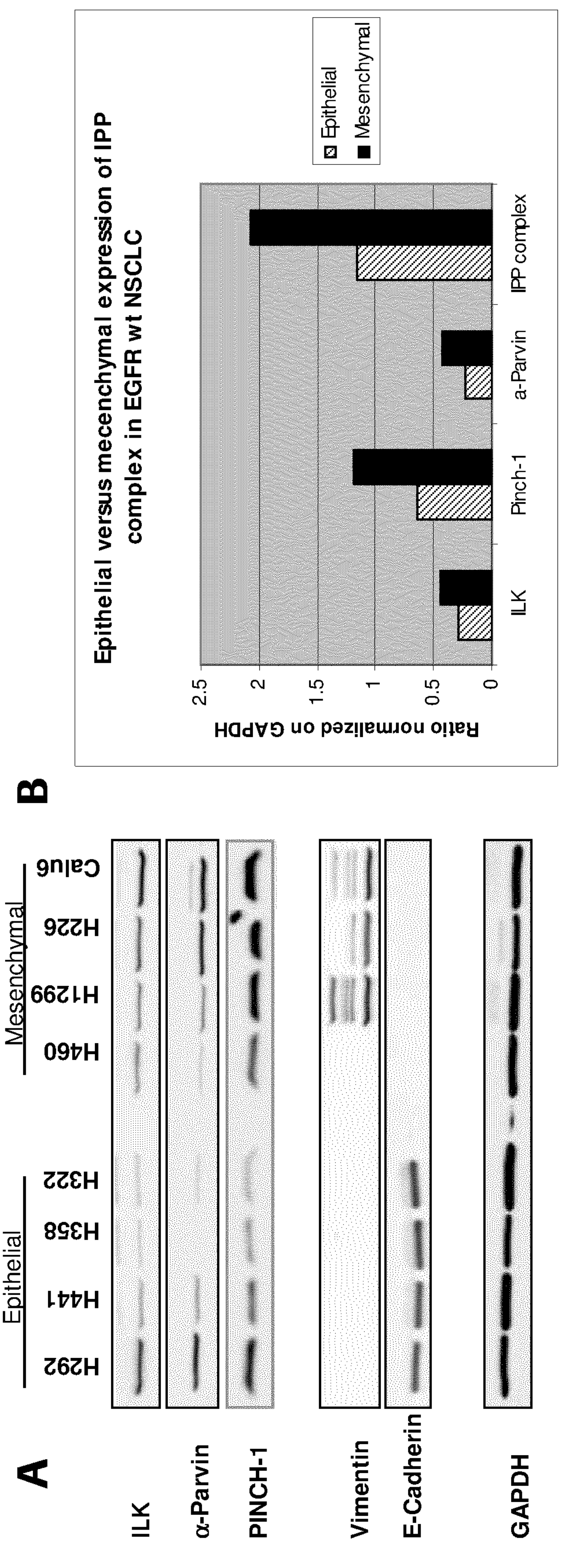
A
Epithelial
Mesenchymal
H292
H441
H358
H322
H460
H1299
H226
Calu6
ILK
α-Parvin
PINCH-1
Vimentin
E-Cadherin
GAPDH
B
Epithelial versus mecenchymal expression of IPP complex in EGFR wt NSCLC
Ratio normalized on GAPDH
2.5
2
1.5
1
0.5
0
ILK
Pinch-1
a-Parvin
IPP complex
Epithelial
Mesenchymal

Figure 6

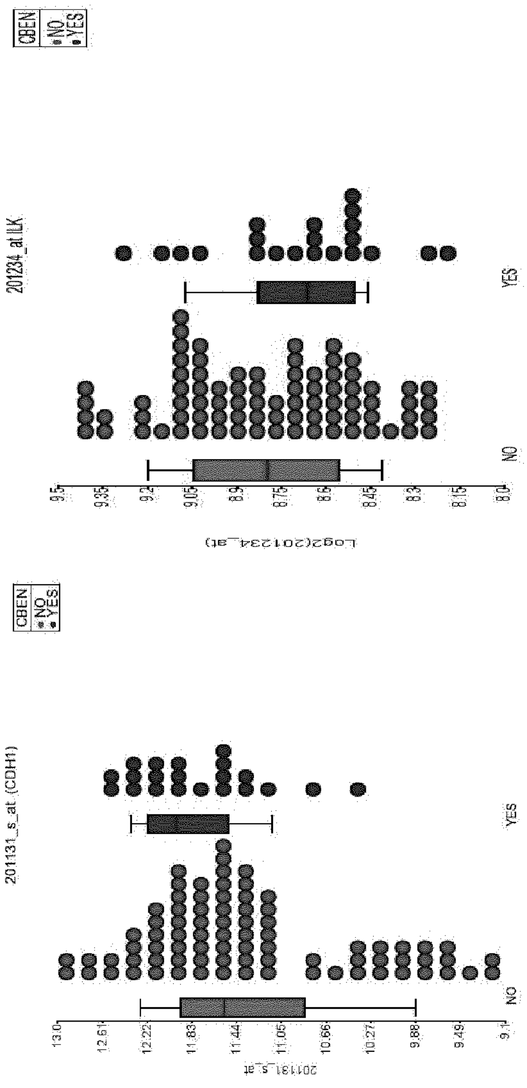
201234_at ILK
CBEN
NO
YES
Log2(201234_at)
9.5
9.35
9.2
9.05
8.9
8.75
8.6
8.45
8.3
8.15
8.0
201131_s_at (CDH1)
201131_s_at
13.0
12.61
12.22
11.83
11.44
11.05
10.66
10.27
9.88
9.49
9.1

Figure 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5202231 A
- US 5445934 A
- US 5525464 A
- US 5695940 A
- US 5744305 A
- US 5795 A
- US 716 A
- US 15800992 B

### Non-patent literature cited in the description


- **JOHNSTON, M.** *Curr. Biol.,* 1998, vol. 8, R171-174
- **IYER VR et al.** *Science,* 1999, vol. 283, 83-87
- **SAMBROOK J et al.** Molecular Cloning: A Laboratory Manual. Cold Spring Harbor Press, 2000
- **SHEVCHENKO, A. ; WILM, M. ; VORM, O. ; MANN, M.** Mass spectrometric sequencing of proteins silver-stained polyacrylamide gels. *Anal Chem,* 1996, vol. 68, 850-58
- **SMYTH, G. K. ; SPEED, T. P.** Normalization of cDNA microarray data. *Methods,* 2003, vol. 31, 265-273
- **SMYTH, G. K. ; MICHAUD, J. ; SCOTT, H.** The use of within-array replicate spots for assessing dierential expression in microarray experiments. *Bioinformatics,* 2005, vol. 21 (9), 2067-2075
- **IRIZARRY RA ; HOBBS B ; COLLIN F ; BEAZER-BARCLAY YD ; ANTONELLIS KJ ; SCHERF U ; SPEED TP.** Exploration, normalization, and summaries of high density oligonucleotide array probe level data. *Biostatistics Apr,* 2003, vol. 4 (2), 249-64
- **HOLDER, D ; RAUBERTAS, RF ; PIKOUNIS, VB ; SVETNIK, V ; SOPER, K.** Statistical analysis of high density oligonucleotide arrays: a SAFER approach. *Proceedings of the ASA Annual Meeting 2001,* 2001
- **BRETZ F ; HOTHORN LA.** Testing dose-response relationships with a priori unknown, possibly nonmonotone shapes. *J Biopharm Stat.,* 2001, vol. 11 (3), 193-207
- **SMYTH GK.** Linear models and empirical bayes methods for assessing differential expression in microarray experiments. *Stat Appl Genet Mol Biol.,* 2004, vol. 3
- **BRETZ F ; HOTHORN LA.** Detecting dose-response using contrasts: asymptotic power and sample size determination for binomial data. *Stat Med. 2002,* 2002, vol. 21 (22), 3325-35
- **P. H. WESTFALL ; S. S. YOUNG.** Resampling-based multiple testing: Examples and methods for p-value adjustment. John Wiley & Sons, 1993, 116-117
- R Development Core Team. *R: A language and environment for statistical computing. R Foundation for Statistical Computing,* 2009, ISBN 3-900051-07-0, http://www.R-project.org
- **GE,Y. et al.** Resampling-based multiple testing for microarray data analysis. *Test,* 2003, vol. 12 (1), 1-77
- **THOMSON et al.** *Cancer Res.,* 2005, vol. 65, 9455-9462
- **TAKANAMI, I.** *BMC Cancer,* 2005, vol. 5, 1-7
- **BERMAN, H. M. ; WESTBROOK, J. ; FENG, Z. ; GILLILAND, G. ; BHAT, T. N. ; WEISSIG, H. ; SHINDYALOV, I. N. ; BOURNE, P. E.** *The Protein Data Bank,* 2000, 235-242
- **STAMOS, J. ; SLIWKOWSKI, M. X. ; EIGENBROT, C.** Structure of the epidermal growth factor receptor kinase domain alone and in complex with a 4-anilinoquinazoline inhibitor. *J Biol Chem,* 2002, vol. 277, 46265-46272
- **YUN, C. H. ; BOGGON, T. J. ; LI, Y. ; WOO, M. S. ; GREULICH, H. ; MEYERSON, M. ; ECK, M. J.** Structures of lung cancer-derived EGFR mutants and inhibitor complexes: mechanism of activation and insights into differential inhibitor sensitivity. *Cancer Cell,* 2007, vol. 11, 217-227
- **FUKUDA, K. ; GUPTA, S. ; CHEN, K. ; WU, C. ; QIN, J.** The pseudoactive site of ILK is essential for its binding to alpha-Parvin and localization to focal adhesions. *Mol Cell,* 2009, vol. 36, 819-830
- **TAN, E. H. ; RAMLAU, R. ; PLUZANSKA, A. ; KUO, H. P. ; RECK, M. ; MILANOWSKI, J. ; AU, J. S. ; FELIP, E. ; YANG, P. C. ; DAMYANOV, D.** A multicentre phase II gene expression profiling study of putative relationships between tumour biomarkers and clinical response with erlotinib in non-small-cell lung cancer. *Ann Oncol,* 2010, vol. 21, 217-222

- **YAUCH, R. L. ; JANUARIO, T. ; EBERHARD, D. A. ; CAVET, G. ; ZHU, W. ; FU, L. ; PHAM, T. Q. ; SORIANO, R. ; STINSON, J. ; SESHAGIRI, S.** Epithelial versus mesenchymal phenotype determines in vitro sensitivity and predicts clinical activity of erlotinib in lung cancer patients. *Clin Cancer Res,* 2005, vol. 11, 8686-8698
- **CABODI, S. ; DEL PILAR CAMACHO-LEAL, M. ; DI STEFANO, P. ; DEFILIPPI, P.** Integrin signalling adaptors: not only figurants in the cancer story. *Nat Rev Cancer,* 2010, vol. 10, 858-870
- **THOMSON, S. ; BUCK, E. ; PETTI, F. ; GRIFFIN, G. ; BROWN, E. ; RAMNARINE, N. ; IWATA, K. K. ; GIBSON, N. ; HALEY, J. D.** Epithelial to mesenchymal transition is a determinant of sensitivity of non-small-cell lung carcinoma cell lines and xenografts to epidermal growth factor receptor inhibition. *Cancer Res,* 2005, vol. 65, 9455-9462
- **THOMSON, S. ; PETTI, F. ; SUJKA-KWOK, I. ; EPSTEIN, D. ; HALEY, J. D.** Kinase switching in mesenchymal-like non-small cell lung cancer lines contributes to EGFR inhibitor resistance through pathway redundancy. *Clin Exp Metastasis,* 2008, vol. 25, 843-854
- **SINGH, A. ; SETTLEMAN, J.** EMT, cancer stem cells and drug resistance: an emerging axis of evil in the war on cancer. *Oncogene,* 2010, vol. 29, 4741-4751
- **OLOUMI, A. ; MCPHEE, T. ; DEDHAR, S.** Regulation of E-cadherin expression and beta-catenin/Tcf transcriptional activity by the integrin-linked kinase. *Biochim Biophys Acta,* 2004, vol. 1691, 1-15

SEQUENCE LISTING

<110> F.Hoffmann-LaRoche AG

<120> IPP complex as marker for erlotinib treatment

<130> 27042

<160> 3

<170> PatentIn version 3.5

<210> 1
<211> 1731
<212> DNA
<213> Homo sapiens

<400> 1

ggatcctgca gcccgagtcc cgtcctcagg cttccccaat ccaggggact cggcgccggg 60
acgctgctat ggacgacatt ttcactcagt gccgggaggg caacgcagtc gccgttcgcc 120
tgtggctgga caacacggag aacgacctca accaggggga cgatcatggc ttctcccccct 180
tgcactgggc ctgccgagag ggccgctctg ctgtggttga gatgttgatc atgcgggggg 240
cacggatcaa tgtaatgaac cgtggggatg acaccccccct gcatctggca gccagtcatg 300
gacaccgtga tattgtacag aagctattgc agtacaaggc agacatcaat gcagtgaatg 360
aacatgggaa tgtgcccctg cactatgcct gtttttgggg ccaagatcaa gtggcagagg 420
acctggtggc aaatggggcc cttgtcagca tctgtaacaa gtatggagag atgcctgtgg 480
acaaagccaa ggcacccctg agagagcttc tccgagagcg ggcagagaag atgggccaga 540
atctcaaccg tattccatac aaggacacat tctggaaggg gaccacccgc actcggcccc 600
gaaatggaac cctgaacaaa cactctggca ttgacttcaa acagcttaac ttcctgacga 660
agctcaacga gaatcactct ggagagctat ggaagggccg ctggcagggc aatgacattg 720
tcgtgaaggt gctgaaggtt cgagactgga gtacaaggaa gagcagggac ttcaatgaag 780
agtgtccccg gctcaggatt ttctcgcatc caaatgtgct cccagtgcta ggtgcctgcc 840
agtctccacc tgctcctcat cctactctca tcacacactg gatgccatat ggatccctct 900
acaatgtact acatgaaggc accaatttcg tcgtggacca gagccaggct gtgaagtttg 960
ctttggacat ggcaaggggc atggccttcc tacacacact agagcccctc atcccacgac 1020
atgcactcaa tagccgtagt gtaatgattg atgaggacat gactgcccga attagcatgg 1080
ctgatgtcaa gttctctttc caatgtcctg gtcgcatgta tgcacctgcc tgggtagccc 1140
ccgaagctct gcagaagaag cctgaagaca caaacagacg ctcagcagac atgtggagtt 1200
ttgcagtgct tctgtgggaa ctggtgacac gggaggtacc ctttgctgac ctctccaata 1260
tggagattgg aatgaaggtg gcattggaag gccttcggcc taccatccca ccaggtattt 1320
cccctcatgt gtgtaagctc atgaagatct gcatgaatga agaccctgca aagcgaccca 1380
aatttgacat gattgtgcct atccttgaga agatgcagga caagtaggac tggaaggtcc 1440
ttgcctgaac tccagaggtg tcgggacatg gttgggggaa tgcacctccc caaagcagca 1500
ggcctctggt tgcctccccc gcctccagtc atggtactac cccagccatg gggtccatcc 1560
ccttccccca tccctaccac tgtggcccca agaggggcgg gctcagagct ttgtcacttg 1620
ccacatggtg tctcccaaca tgggagggat cagccccgcc tgtcacaata aagtttatta 1680
tgaaaaaaaa aaaaaaaaaa aaaaaaaaaa aaaaaaaaaa aaaaaaaaaa a 1731

<210> 2
<211> 4091

<212> DNA
<213> Homo sapiens

<400> 2

```
gaaagccgca gcctcagtcc cgccgccgcc cgctgcgtcc gcccagcgcc agctccgcgt      60
cccgaccggc ccgcggcagc ctgcgccgcg ccatggccac ctccccgcag aagtcgcctt     120
ctgtccccaa gtctcccact cccaagtcgc ccccgtcccg caagaaagat gattccttct     180
tggggaaact cggagggacc ctggcccgga ggaagaaagc caaggaggtg tccgagctgc     240
aggaggaggg aatgaacgcc atcaacctgc ccctcagccc aattcccttt gagctggacc     300
ccgaggacac gatgctggag gagaatgagg tgcgaacaat ggtggatcca aactcacgca     360
gtgaccccaa gcttcaagaa ctgatgaagg tattaattga ctggattaat gatgtgttgg     420
ttggagaaag aatcattgtg aaagacctag ctgaagattt gtatgatgga caagtcctgc     480
agaagctttt cgagaaactg gagagtgaga agctaaatgt ggctgaggtc acccagtcag     540
agattgctca gaagcaaaaa ctgcagactg tcctggagaa gatcaatgaa accctgaaac     600
ttcctcccag gagcatcaag tggaatgtgg attctgttca tgccaagagc ctggtggcca     660
tcttacacct gctcgttgct ctgtctcagt atttccgcgc accaattcga ctcccagacc     720
atgtttccat ccaagtggtt gtggtccaga aacgagaagg aatcctccag tctcggcaaa     780
tccaagagga aataactggt aacacagagg ctctttccgg gaggcatgaa cgtgatgcct     840
ttgacacctt gttcgaccat gccccagaca agctgaatgt ggtgaaaaag acactcatca     900
ctttcgtgaa caagcacctg aataaactga acctggaggt cacagaactg gaaacccagt     960
ttgcagatgg ggtgtacctg gtgctgctca tggggctcct ggagggctac tttgtgcccc    1020
tgcacagctt cttcctgacc ccggacagct ttgaacagaa ggtcttgaat gtctcctttg    1080
cctttgagct catgcaagat ggagggttgg aaaagccaaa accgcggcca gaagacatag    1140
tcaactgtga cctgaaatct acactacgag tgttgtacaa cctcttcacc aagtaccgta    1200
acgtggagtg aggggctgcc ctgggcccac cactgcccaa gagttcttgc tgttggcgta    1260
ctggaccctc ctccgaactg ccttaccctg cttattcctg tctcttgcac tgtgctctcc    1320
```

cacaagtcca gctgcaaccc agagatagtg gaaactgaaa ttaggaagga aatcatcaat 1380
aactcagtgg gctgacccat ccctcccagg cgctggggac caacctagca atgaaggttg 1440
ggaaggttgt tcccttcccg gtgccaggtc cagatttccc tccatgattt gggaaccagc 1500
ttaggcaaaa gagtccccac aagatgaaaa taaagatcct agttaccatt caaaggatgc 1560
taactgtgtg tcaggcccca cactaagtgc tctgctctga tatactcaag gccattaatc 1620
ttcaggactc ccattgacgt aggtgtttca ttcccctttt acagatgagg aaactaaggc 1680
ttggaggtta aatgacttgc cagaagttgg aatttttttc ctctttgaac ataacctctc 1740
ccttctccct aaaggtaacc actattctga gtccaatcat caaggttttg cttttctttt 1800
tagctaagta tgcattcctc aatagtagac agtacaacat gtttataaca agccaattac 1860
attatgttct ttgcatgttc taaagttgtg tatgtgtgtg cacatctgag cacgtgcaca 1920
tgtacacctg agccaaaaac acgagaaccc actgatctca ccactggggc aagctaggtc 1980
agagcttagt gattcacact gaaattggca aattggattt aacccaatta atagtgtgtg 2040
tgtggcagga gtcatgtccc tcacatcctt tgtacaaatg aaaattactc ttaattcctt 2100
cagatttata ataactctgt actttggttt cagggtgaca tttgggaagg attttgttta 2160
gaattaatgg agtggcacat tttgcagcct ttttgcttga ttgcatgtaa tggaaatgcc 2220
ctatattttc ctgcaaaata agtactaaat tcattatcgt taagcaaatg tacaatatgc 2280
tcaggcaccg cagagagctg ggcacgggcc catgtgagca tcactttgga agtagggctc 2340
ttcaacaggg acccttgaac tttaaagaaa ggaacttctt tttgccttct aattgatcat 2400
ttagactatt ctggctaagt ctgcccacat gtaattaccg gctaattcaa gcgaggaaaa 2460
atgtaagtca tttagaccaa agccaagcag tttctttgcg tgggttactc aagggcttgt 2520
ggttacttgt atctcctcta tgtgaacttg actttgaaag acagagctct agtgtgccag 2580
cctgctaagt cctgtaagaa tagggaaggg cggagggggg tgggcagtga ctaggggacg 2640
agaagcatgg ggaaaatatt tgcactctaa acatacagag atagaggtgg ggcttgtggt 2700
acttaacact tgtagccatc aactgactga gaccttgggc taaataatca attgtgctga 2760
tattacatct cgttatggaa tgttcctaaa tatgccaggt agacaccagc ccaagtaccc 2820
tcctccagaa gtctgtgact accttgtcac tactttaggc ccattccaca aagcccatct 2880
ctggtttgag aattcatttt gatctgtatc tacaccaccc aaagttaggc ctcctataat 2940
gtccaaaaca ttcctttcag cctttttatt tcttactgta ctgtctctta ctgtactgtc 3000
tatctgcagt aattgaggac ccataaaatt tagataacta catgtctttg ctcttagaat 3060
tgtcactcag cataatgagc atttaacata caaaggcaat gtactgtttt gtgttgatct 3120
atgtaaaaga atacaattct tttttacata attagtgaaa ttttattttt tattaggaaa 3180

cactaaatag tgtaatattt cttttgcttt taaaaaaatt cctggtagca aatcaagata 3240
aataattgct tcattttctt gagcaatact gaagcaggat gaagtaagag gaatgcattc 3300
atttaaacat gctttgcttt atgaattttg tctctttttt ggtctctttt tcttatattc 3360
aagttacaaa tgtacaagta tccttactaa gagtgctcct tttgtatttt acatatatac 3420
agtatgaaaa tacattggaa cactaggaaa gtttttaaat aacagttcta atttatcaga 3480
aaattgtgtt ttgggattga gttctttgtc tcagcccaga atcccaggtc ctgggcctgg 3540
ttttctaatg ctgtcatctc agttcgatat tttactttag aatctggaat ctcctactta 3600
atatatgacc atgactttga aaggcaaaag aggaatcaag aataaataaa acaaacttaa 3660
tcttcatctt taaaaaaaag aaaaaagaaa ccaaaatgag aatcaacact tcataggctc 3720
actgggtttt cttttctttt ctgttaattt aaactcagtt atttttaatg cttaatacat 3780
acatggtgca aaatttaaaa agcgcaaata ggtatctagt ggaaaaccta agcctccctc 3840
tctctccggc acccattacc tctccctgga ggcaactgtt ttgatccatt tcttacacac 3900
actgccagag atactctagg catgtaaagc acaaacatac atataaaatc tgcgggcttc 3960
aaaaaatata agtaggatgt catctatact gtcatacact ttgtttttta tcacttactt 4020
aatgttatat cttggatatt gtattaccct gggtattaaa aagaactcct ttcacatttt 4080
aaaataaaaa a 4091

<210> 3
<211> 1877
<212> DNA
<213> Homo sapiens

<400> 3

```
acaaagactg tgtcactact ttgtggaatg ttattttaga acttccagaa tgcctcagta      60
aatcctgact gccatgtaac tgtgtactgt gctgctgagc tcttaacatg aagcagcagc     120
agtgaccctg gcgggccagg ctgacgtgcg tgggaacagt gggagtgacc ctggctggga     180
agcagggagg cctggatatt gtttctcctg gctctgtgtc ctgccattct catccctgtg     240
ctcagagctc tcaagcaccc acgatggcct tctcaggccg agcgcgcccc tgcattatcc     300
cagagaacga agaaatcccc cgagcagccc ttaacactgt ccacgaggcc aatgggaccg     360
aggacgagag ggctgtttcc aaactgcagc gcaggcacag tgacgtgaaa gtctacaagg     420
agttctgtga cttttatgcg aaattcaaca tggccaacgc cctggccagc gccacttgcg     480
agcgctgcaa gggcggcttt gcgcccgctg agaagatcgt gaacagtaat ggggagctgt     540
accatgagca gtgtttcgtg tgcgctcagt gcttccagca gttcccagaa ggactcttct     600
atgagtttga aggaagaaag tactgtgaac atgactttca gatgctcttt gccccttgct     660
gtcatcagtg tggtgaattc atcattggcc gagttatcaa agccatgaat aacagctggc     720
```

```
atccggagtg cttccgctgt gacctctgcc aggaagttct ggcagatatc gggtttgtca      780
agaatgctgg gagacacctg tgtcgcccct gtcataatcg tgagaaagcc agaggccttg      840
ggaaatacat ctgccagaaa tgccatgcta tcatcgatga gcagcctctg atattcaaga      900
acgaccccta ccatccagac catttcaact gcgccaactg cgggaaggag ctgactgccg      960
atgcacggga gctgaaaggg gagctatact gcctcccatg ccatgataaa atgggggtcc     1020
ccatctgtgg tgcttgccga cggcccatcg aagggcgcgt ggtgaacgct atgggcaagc     1080
agtggcatgt ggagcatttt gtttgtgcca agtgtgagaa accctttctt ggacatcgcc     1140
attatgagag gaaaggcctg gcatattgtg aaactcacta taaccagcta tttggtgatg     1200
tttgcttcca ctgcaatcgt gttatagaag gtgatgtggt ctctgctctt aataaggcct     1260
ggtgcgtgaa ctgctttgcc tgttctacct gcaacactaa attaacactc aagaataagt     1320
ttgtggagtt tgacatgaag ccagtctgta agaagtgcta tgagaaattt ccattggagc     1380
tgaagaaaag acttaagaaa ctagctgaga ccttaggaag gaaataagtt cctttatttt     1440
ttcttttcta tgcaagataa gagattacca acattacttg tcttgatcta cccatattta     1500
aagctatatc tcaaagcagt tgagagaaga ggacctatat gaatggtttt atgtcatttt     1560
tttaattaaa aaagaaaaat tcatataatc gtgtttaaaa cacaaatgaa gtcagtattt     1620
gcctttgtta acccttatcc atttgttgac atgtagactg tttacaaaaa aaaaacacat     1680
ggttaaatgt taaattttaa ttaaggcccc caaaaattaa atataacttt ttaaaatgaa     1740
aggagtcacc ttttacatga ctcaggtgaa aaaacagtat aaacattaat ttactttgtg     1800
ttcaaaagaa aattccaact gctgttgggg aaggacacag aaaagaaaaa taaccaccca     1860
agaaaaacaa aaaaaaa                                                    1877
```